# FASCICULE DE BREVET EUROPEEN

(11) **EP 2 167 967 B1**
(45) Date de publication et mention de la délivrance du brevet: **27.05.2015**
(21) Numéro de dépôt: 08806156.9
(22) Date de dépôt: 02.07.2008
(51) Int. Cl.: G01N 33/543, G01N 33/545, G01N 33/80

(54) **DISPOSITIF ET PROCEDE D'IDENTIFICATION ET DE DETERMINATION DE GROUPES SANGUINS**
VORRICHTUNG UND VERFAHREN ZUR IDENTIFIKATION UND BESTIMMUNG VON BLUTGRUPPEN
DEVICE AND METHOD FOR IDENTIFYING AND DETERMINING BLOOD GROUPS

(30) Priorité: 02.07.2007 FR 0704741
(43) Date de publication de la demande: 31.03.2010
(73) Titulaire: ABO DIAG, 33650 Martillac (FR)
(72) Inventeur: CHAÏBI, Najim, 33000 Bordeaux (FR)
(74) Mandataire: Regimbeau
(86) Numéro de dépôt international: PCT/FR2008/051232
(87) Numéro de publication internationale: WO 2009/007649

(56) Documents cités:
- WO-A-2004/103939
- WO-A-2006/098803
- US-A- 5 096 809
- US-A- 5 126 276
- US-A- 5 710 049
- US-A1- 2004 096 356
- US-A1- 2010 041 565
- KNIGHT R C ET AL: "Detection of red cell antibodies: Current and future techniques" BRITISH JOURNAL OF BIOMEDICAL SCIENCE, ROYAL SOCIETY OF MEDICINE SERVICES, LONDON, GB, vol. 52, no. 4, 1995, pages 297-305, XP008007278 ISSN: 0967-4845

## Description

La présente invention est relative à un dispositif pour l'identification et la détermination d'antigènes érythrocytaires de groupes sanguins et des anticorps plasmatiques correspondants à partir d'un échantillon de sang total.

L'invention vise également l'utilisation de ce dispositif, un procédé mettant en oeuvre ce dispositif, ainsi qu'un kit de détermination des groupes sanguins.

Le sang est un tissu conjonctif liquide présent chez l'homme et la plupart des animaux évolués. Malgré une composition cellulaire identique, il existe une variabilité des divers éléments du sang, définis par différents systèmes antigéniques appelés groupes sanguins.

En pratique on s'intéresse plus particulièrement aux groupes sanguins érythrocytaires, systèmes d'antigènes situés à la surface des globules rouges, tels que par exemple les systèmes ABO, Rhésus, Kell, Duffy, MNS, Lewis, etc. Classiquement, la détermination d'un groupe sanguin se fait sur la reconnaissance antigène-anticorps. Lorsqu'un anticorps spécifique de l'antigène reconnaît celui-ci, il se fixe. Généralement les anticorps utilisés dans la reconnaissance de groupes sanguins sont des immunoglobulines IgM agglutinant les globules rouges. Les techniques habituellement utilisées, consistent à rechercher et identifier la présence ou l'absence d'antigènes de groupe sanguin à la surface des érythrocytes ou à rechercher et identifier la présence ou l'absence d'anticorps anti-antigène de groupe sanguin dans le plasma.

En particulier, pour le système ABO, l'épreuve de Beth-vincent permet de déterminer les antigènes portés par les globules rouges, et l'épreuve complémentaire de Simonin-Michon ou contre épreuve-sérique permet de déterminer les anticorps circulant dans le sérum.

Dans l'épreuve de Beth-Vincent, les globules rouges de l'individu, obtenus après séparation de phase des cellules et du plasma, soit par centrifugation soit par décantation, sont mis en présence de réactifs d'anticorps de spécificité connue. Généralement ce test est rendu visible par observation d'agglutination des globules rouges lorsque les anticorps reconnaissent les antigènes érythrocytaires correspondants.

Dans l'épreuve de Simonin, le plasma de l'individu est mis en présence de globules rouges tests appartenant chacun à un groupe antigénique précis du système ABO. Il s'agit d'un test d'agglutination du plasma de l'individu avec des hématies tests.

Pour la recherche d'anticorps dits irréguliers ou RAI, il s'agit de détecter la présence ou l'absence dans le sang d'un individu d'immunoglobulines dirigées contre divers antigènes érythrocytaires de l'individu. Pour cela on cherche à mettre en évidence la fixation de ces immunoglobulines sur des globules rouges tests dont les antigènes sont connus, avec la technique de Coombs direct et indirect, la comparaison des résultats permettant de déduire la présence ou l'absence d'immunoglobulines.

Il existe un grand nombre de procédés et de dispositifs utilisés pour le phénotypage dans le domaine de l'immunologie-hématologie, les techniques pouvant être manuelles, sur plaque d'opaline, en tube ou en cupule de microplaque, ou encore complètement automatisées à l'aide de robot distributeur d'échantillon et de réactif, agitateur, incubateur et lecteur automatique. On connaît notamment deux techniques de référence : les techniques en microplaques et les techniques en filtration par gel-test.

Toutefois ces techniques existantes de phénotypage de groupes sanguins présentent de nombreux inconvénients.

Les techniques en microplaque par exemple nécessitent une phase d'agitation qui est critique car les multiples réactions simultanément présentes sur le support n'ont pas la même cinétique de remise en suspension. Elles doivent être réalisées sous contrôle visuel et il faut être particulièrement attentif aux phénomènes d'adhérence de certains réactifs.

De même, lors de la mise en oeuvre de techniques en filtration par gel-test, il existe également un risque de non détection de certaines agglutinations notamment lors de l'épreuve plasmatique du groupe ABO. Un autre inconvénient est la détection trop fréquente d'auto-anticorps en relation avec les préparations d'hématies-tests, en particulier celles traitées par des enzymes protéolytiques.

En outre, toutes ces techniques présentent un inconvénient majeur du fait qu'elles nécessitent une centrifugation préalable du sang total afin de séparer les éléments constitutifs du sang, étape contraignante qui augmente fortement le temps et le coût d'analyse, et qui nécessite l'utilisation de centrifugeuses encombrantes et difficiles à manipuler.

Afin de supprimer cette étape lourde de centrifugation, des variantes ont été développées, basées sur l'utilisation de particules magnétiques.

On peut citer à titre d'exemple la demande de brevet EP-0.351.857 qui décrit un procédé de dosage immunologique utilisant des marqueurs magnétisés tels que des anticorps ou des antigènes fixés sur des billes de latex magnétiques. Il est notamment décrit une technique RAI par immunoadhérence dans laquelle on applique un champ magnétique sur des hématies préalablement fixées au fond d'une cupule de microplaque, sensibilisées avec le sérum à tester, lavées et mélangées à des billes de latex magnétiques revêtues d'une anti-immunoglobuline. On connaît également la demande EP-0.230.768 qui décrit un procédé de co-agrégation de particules magnétiques capables de se lier à une substance contenue dans un échantillon au moyen de composés polycationiques en présence d'un champ magnétique.

Toutefois ces différentes techniques présentent également de nombreux inconvénients. Elles sont difficiles et longues à mettre en oeuvre, peu économiques et nécessitent l'utilisation de dispositifs complexes et peu mobiles. Le brevet US-5126 276 décrit un dispositif de détection d'analytes dans un fluide biologique, comprenant un support solide constitué par une carte solide avec plusieurs languettes planes à la périphérie de la carte, dans lequel au moins les languettes comprennent une zone réactive.

Le brevet US-5 710 049 décrit un dispositif d'analyse d'échantillons avec un traceur métallique. Le document US 2004/0096356 décrit un dispositif permettant la détection d'analytes de produits laitiers.

WO 2006/098 803 décrit un système pour détecter la présence d'un analyte dans un fluide.

GB2250342 décrit un dispositif de détermination de groupe sanguin, comprenant des zones réactives, dans lequel la zone de dépôt de l'échantillon est distincte des zones réactives et de la zone où on lit le résultat.

Aussi, la présente invention vise à pallier les inconvénients de l'art antérieur en proposant un moyen simple et efficace pour le phénotypage de groupes sanguins érythrocytaires, et l'identification et la détermination des anticorps plasmatiques correspondants, présentant une précision et une exactitude au moins comparables à celles obtenues par les méthodes de référence.

En particulier l'objectif de l'invention est de proposer un système économique, facile d'utilisation, automatisable et mobile, capable de déterminer et de détecter rapidement des groupes sanguins érythrocytaires par immobilisation des globules rouges directement à partir d'un échantillon de sang total, sans utilisation d'un appareillage de centrifugation et/ou d'un appareil de mesure quelconque.

Pour répondre à cet objectif, la présente invention propose un dispositif pour l'identification et la détermination de groupes sanguins à partir d'un échantillon de sang total, comprenant un support solide qui comporte au moins une zone réactive sur laquelle le sang est déposé et sur laquelle le résultat est lu, ladite zone réactive étant constituée par au moins une membrane poreuse polymère en polyéthylène haute densité, présentant des pores de diamètre compris entre 1 et 20 µm, imprégnée par des réactifs anticorps monoclonaux ou des réactifs antiglobulines permettant l'immobilisation des anticorps plasmatiques, et une membrane absorbante, destinée à récupérer l'excès de sang de l'échantillon analysé. Le dispositif permet d'identifier et de déterminer des antigènes érythrocytaires de groupes sanguins et/ou des anticorps plasmatiques correspondants.

L'invention vise également l'utilisation de ce dispositif, ainsi qu'un procédé de phénotypage de groupes sanguins érythrocytaires par réaction d'immobilisation des globules rouges, mettant en oeuvre ce dispositif.

Avantageusement la présente invention permet de détecter et/ou de déterminer un groupe sanguin érythrocytaire rapidement, directement à partir de sang total, par un résultat de lecture par positivité, sans utiliser de centrifugeuse ou d'appareillage de mesure. Le dispositif permet une détection simultanée des antigènes de groupes sanguins et des anticorps plasmatiques correspondants. Selon un dernier aspect, l'invention est également relative à un kit pour l'identification et la détermination d'antigènes érythrocytaires de groupes sanguins et des anticorps plasmatiques correspondants.

D'autres caractéristiques et avantages ressortiront de la description qui va suivre de l'invention, description donnée à titre d'exemple uniquement, en regard des dessins annexés sur lesquels :
- la figure 1 représente un schéma du dispositif selon l'invention vu en perspective,
- la figure 2a représente une coupe schématique d'un premier mode de réalisation d'une zone réactive d'un dispositif selon l'invention,
- la figure 2b représente une coupe schématique d'un second mode de réalisation d'un dispositif selon l'invention, et
- figure 3 représente une vue en perspective d'un mode de réalisation du dispositif selon l'invention.

Le dispositif selon l'invention est destiné à la détermination et/ou la détection de groupes sanguins érythrocytaires à partir d'un échantillon de sang total. Comme représenté sur les figures, il comprend un support solide 1 comportant au moins une zone réactive sur laquelle le sang est déposé et sur laquelle le résultat est lu 4, ladite zone réactive étant constituée par au moins :
- une membrane poreuse 3 polymère en polyéthylène haute densité, présentant des pores de diamètre compris entre 1 et 20 µm, imprégnée par des anticorps monoclonaux ou des réactifs antiglobulines permettant l'immobilisation des anticorps plasmatiques, et
- une membrane absorbante 2, destinée à récupérer l'excès de sang de l'échantillon analysé.

La membrane poreuse polymère 3 présente des pores de diamètre compris entre 1 et 20 µm, préférentiellement entre 1 et 14 µm.

Selon un mode de réalisation préféré la membrane poreuse 3 est transparente. Il s'agit d'une membrane en polyéthylène haute densité, qui présente des caractéristiques particulièrement adaptées à l'invention en particulier pour l'activation et l'immobilisation des anticorps. Encore plus préférentiellement, la membrane poreuse 3 est telle qu'elle présente une bonne résistance, une vitesse de migration rapide correspondant à l'adsorption d'une goutte de sang total en moins de 10 secondes, et une porosité moyenne de 7µm.

La membrane 3 est imprégnée par des réactifs anticorps monoclonaux ou des réactifs antiglobulines permettant l'immobilisation des anticorps plasmatiques. A titre d'exemple, dans le cas où le dispositif est utilisé pour une épreuve de Beth-Vincent, la membrane 3 est imprégnée de réactifs anticorps monoclonaux, tels que des anticorps anti-A1, anti-A2, anti-B, anti-AB ou anti-D; dans le cas où le dispositif est utilisé pour une épreuve de Simonin, la membrane 3 est imprégnée de réactifs antiglobulines.

Les réactifs de complexation sont liés de manière spécifique sur la zone d'activité.

Les réactifs anticorps monoclonaux ou les réactifs antigloblines sont fixés sur la membrane 3 par tout moyen approprié. Notamment ils peuvent être fixés par adsorption passive et sélective. Cette sélectivité améliore la justesse et la précision du test.

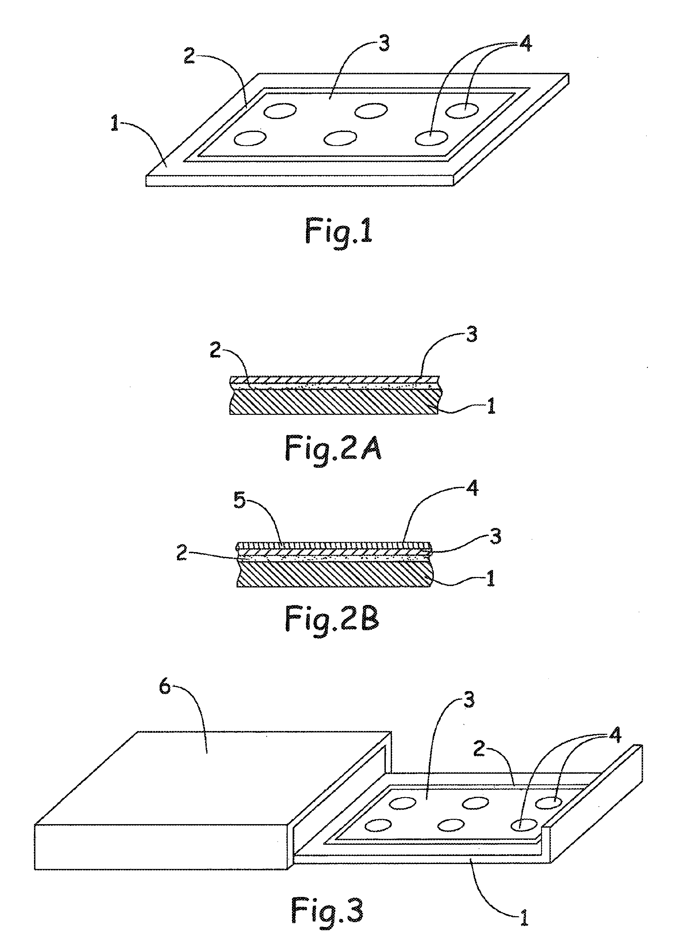

Selon une variante illustrée sur la figure 2b, la ou les zones réactives 4 du dispositif comprennent également un filtre 5. Ce filtre peut par exemple être constitué de fibres de verre de diamètre compris entre 1 et 7µm.

Lorsque le dispositif est utilisé pour l'identification et la détermination d'anticorps dans le plasma de l'individu, ce filtre 5 est préférentiellement imprégné par des réactifs capables d'agglutiner les érythrocytes, de façon à retenir les globules rouges et à ne laisser passer que le plasma lorsque du sang total est déposé. A titre d'exemple, ces réactifs peuvent être des agglutines de soja, lectines ou tout autre substance biologique ou non permettant d'agglutiner des globules rouges.

Ces réactifs d'agglutination sont fixés sur le filtre 5 par tout moyen approprié. Notamment ils peuvent être fixés par adsorption.

Selon un mode de réalisation particulier de l'invention représenté sur la figure 3, le dispositif comprend également un réservoir 6 contenant une solution de lavage et/ou de révélation. Ce réservoir peut être fixé de manière solidaire au dispositif.

Le dispositif selon l'invention peut être utilisé pour déterminer et/ou détecter des groupes sanguins, en particulier des groupes sanguins ABO, Rhésus et RAI, à partir d'un échantillon de sang total.

Le dispositif selon l'invention peut être utilisé en particulier pour la mise en oeuvre d'un procédé de phénotypage de groupes sanguins érythrocytaires par réaction d'immobilisation d'érythrocytes, comprenant les étapes suivantes :
- déposer un échantillon de sang total sur une zone réactive 4,
- laisser réagir au moins 30 secondes, et
- appliquer une solution de lavage afin d'éluer les éléments non liés à la membrane poreuse 3.

Quand le sang est déposé sur la zone réactive 4, les éléments du sang capables de se lier spécifiquement aux réactifs anticorps monoclonaux ou aux réactifs antiglobines de la membrane poreuse 3 sont captés sur ladite membrane. Lorsque la solution de lavage est appliquée, telle qu'une solution de lavage PBS-tween, les éléments du sang non fixés sur la membrane 3 sont éliminés vers la membrane tampon 2.

La lecture du résultat se fait par positivité de manière macroscopique. Si des hématies sont immobilisées sur la membrane poreuse 3, le résultat se traduit par une coloration de la zone réactive 4.

Selon un mode de réalisation de l'invention particulièrement adapté au phénotypage de groupes sanguins ABO, Rhésus et RAI, la membrane 3 est imprégnée par des réactifs anticorps anti-antigènes spécifiques des érythrocytes, tels que par exemple que des anticorps anti-A1, anti-A2, anti-B, anti-AB ou anti-D. La membrane 3 joue alors le rôle d'un piège pour les globules rouges permettant leur fixation sur les immunoglobulines fixées au préalable sur la zone réactive. Les réactifs anticorps liés de manière spécifique sur la zone réactive permettent une captation des globules rouges qui entraîne une immobilisation de ces derniers. En outre, ce phénomène est amplifié par une agglutination de fait, due aux quelques résidus d'anticorps non élués par la solution de lavage.

Lorsque les globules rouges de l'échantillon sanguin déposé sur la zone réactive portent l'antigènes correspondant à l'anticorps présent dans la zone réactive, il y a une réaction de captation et d'immobilisation des globules rouges qui se traduit visuellement par une coloration rouge de la zone réactive.

Lorsque les globules rouges ne sont pas reconnus par les anticorps correspondants, ils sont élués vers la membrane tampon 2 et il y a une absence de coloration sur la zone réactive.

Avantageusement ce procédé n'utilise pas de technique de coloration pour la révélation et la lecture du résultat, ni de dispositif particulier pour observer une agglutination.

En outre, le procédé selon l'invention permet d'éviter des pseudos agglutinations source d'erreurs notamment lors des pratiques en gel-test de l'art antérieur. Selon un autre mode de réalisation de l'invention particulièrement adapté au phénotypage de groupes ABO, Rhésus et RAI, la membrane 3 est imprégnée par des réactifs antiglobulines permettant l'immobilisation de tous les anticorps plasmatiques de l'individu.

Le sang total est déposé sur une zone réactive 4 munie d'un filtre 5 imprégné par des réactifs d'agglutination. Les hématies s'agglutinent au niveau de ce filtre et seul le plasma atteint la membrane poreuse 3.

La membrane 3 joue alors le rôle d'un piège pour les anticorps plasmatiques circulants qui se lient aux réactifs antiglobulines fixés au préalable.

On dépose ensuite des hématies tests d'antigénicité sur la membrane 3, on laisse agir au moins 30 secondes et on applique une solution de lavage afin d'éluer les éléments non liés à la membrane poreuse 3.

Lorsque les anticorps plasmatiques de l'échantillon sanguin liés à la membrane 3 par fixation aux antiglobulines, reconnaissent les hématies-tests correspondant, ces dernières se fixent et s'immobilisent au niveau de la membrane poreuse 3. Le résultat se traduit visuellement par une coloration liée à l'immobilisation des hématies tests appliquées.

Lorsque les anticorps plasmatiques immobilisés dans le support ne reconnaissent pas les hématies-tests, ces dernières sont éliminées par la solution de lavage vers la membrane tampon.

Préférentiellement, afin d'augmenter la sensibilité du test, les solutions d'hématies peuvent être reconcentrées.

Avantageusement le dispositif et le procédé selon l'invention sont fiables, faciles d'utilisation et permettent un phénotypage de groupe sanguin directement à partir d'un échantillon de sang total sans préparation préalable. Le dispositif est aisément transportable et l'utilisateur peut mettre en oeuvre le procédé dans n'importe quel lieu, sans appareillage de mesure ni appareillage de centrifugation. Les exemples qui suivent montrent un mode de réalisation possible du dispositif selon l'invention ainsi que son utilisation pour deux épreuves complémentaires de détermination de groupe sanguin : une épreuve globulaire et pour une épreuve sérique.

### Exemple 1 : Mode de réalisation d'un dispositif selon l'invention

### Membrane poreuse

La membrane poreuse 3 est une membrane polyéthylène haute densité présentant un volume des pores de 40 à 45%, un diamètre de pores moyen de 7µm, une porosité distribuée entre 1 et 14 µm.

Elle est délimitée sous forme de disques de 6mm de diamètre et de 1mm d'épaisseur.

Les disques sont lavés avec de l'éthanol pur afin d'enlever toutes les impuretés de bas poids moléculaire et sont mis à sécher dans une étuve à 60°C.

### Filtre

La membrane filtre 5 est constituée notamment de fibres de verre de diamètre de 1 à 7 µm.

Elle est délimitée sous forme de disques de 6mm de diamètre

### Membrane absorbante

La membrane absorbante 2 est une membrane obtenues chez Whatman avec une capacité s'absorption de 198 mg/cm2.

Elle est délimitée sous forme de disques de 6mm de diamètre et un volume d'absorption équivalent à 50 µl de sang total.

### Support solide

Le support solide 1 permettant d'accueillir les différentes membranes, est un support en plastique de 12cm de longueur et de 8 cm de largeur.

Il est destiné à contenir 72 zones réactives disposées en 8 colonnes, avec chaque zone comprenant un filtre 5, une membrane 3 et une membrane 2. Les colonnes sont destinées à l'identification et la détermination :
- des antigènes A1 pour la colonne 1,
- des antigènes B pour la colonne 2,
- des antigènes AB pour la colonne 3,
- des antigènes D pour la colonne 5,
- des anticorps plasmatiques anti-A pour la colonne 6,
- des anticorps plasmatiques anti-B pour la colonne 7.

Les colonnes 4 et 8 sont des contrôles positifs.

### Réactifs anticorps

Les réactifs anticorps anti-A1, anti-B, anti-AB et anti-D sont des réactifs anticorps monoclonaux purifiés.

Ils sont contenus dans une solution tampon PBS.

Ils sont ensuite déposés sur les disques de membrane 3 destinés à être disposés au niveau de la colonne 1 pour anti-A1, de la colonne 2 pour anti-B, de la colonne 3 pour anti-AB et de la colonne 5 pour anti-D, puis sont mis à sécher.

Un solution de lavage contenant du PBS tween est appliquée sur chaque zone réactive afin d'éliminer les anticorps non lier à la membrane 3.

### Réactifs antiglobulines

Les réactifs antiglobulines sont des réactifs anticorps anti-fc purifiés.

Les réactifs antiglobulines sont ensuite déposés sur les disques de membrane 3 destinés à être disposés au niveau de la colonne 6 et de la colonne 7, puis sont mis à sécher.

Un solution de lavage contenant du PBS tween est appliquée sur chaque zone réactive afin d'éliminer les antiglobulines non liées spécifiquement à la membrane 3.

### Réactifs agglutinant les globules rouges

Des agglutines de soja sont préparées à raison de 90mg/L dans du tampon TRIS à pH 7.

Elles sont ensuite déposées et imprégnées sur le filtre 5 uniquement au niveau de la colonne 6 et de la colonne 7, puis sont mis à sécher.

### Exemple 2 : Mise en oeuvre du procédé pour les colonnes 1 à 5

Une goutte de sang total est déposée sur chaque zone réactive.

On attend entre 30 secondes et une minute, puis on applique une solution de lavage pour éluer les éléments du sang non liés spécifiquement.

Lorsque l'échantillon est déposé sur les zones réactives des colonnes 1 à 5, tous les éléments du sang traversent le filtre 5 pour arriver au niveau de la membrane poreuse 3, car la membrane 5 est dépourvue de réactifs d'agglutination des globules rouges. Au niveau de la membrane poreuse 3 si les globules rouges de l'échantillon sanguin portent l'antigène correspondant à l'anticorps présent dans la zone réactive, il y a une réaction de captation et d'immobilisation des globules rouges qui se traduit par une coloration rouge de la zone réactive. Lorsque les globules rouges ne sont pas reconnus ils sont élués vers la membrane tampon 2, ce qui se traduit visuellement par une absence de coloration de la zone réactive.

### Exemple 3 : Mise en oeuvre du procédé pour les colonnes 6 à 8

Une goutte de sang total est déposée sur chaque zone réactive des colonnes 6 à 8.

Les globules rouges s'agglutinent au niveau du filtre 5 et une certaine quantité de plasma traverse le filtre et arrive au niveau de la membrane poreuse 3 imprégnée d'antiglobulines.

Des hématies tests d'antigénicités connus sont ensuite déposées sur chaque zone réactive.

On attend entre 30 secondes et une minute, puis on applique une solution de lavage pour éluer les éléments du sang non liés spécifiquement.

Lorsque les anticorps plasmatiques de l'échantillon sanguin reconnaissent les hématies-tests correspondantes, ces dernières se fixent au niveau de la zone réactive. Le résultat se traduit par une coloration de la zone réactive.

Lorsque les anticorps plasmatiques immobilisés ne reconnaissent pas les hématies-tests, ces dernières sont éliminées par la solution de lavage vers la membrane tampon 2.

Selon un autre mode de réalisation les hématies tests sont incorporées à la membrane poreuse 3 et maintenues humide par un dispositif de protection contre la déshydratation. Lorsque l'on applique du sang total au niveau d'une zone réactive, les anticorps plasmatiques circulant qui passent à travers le filtre 5 s'immobilisent sur les antigènes membranaires correspondants au niveau de la membrane 3 ou sont élués par la solution de lavage.

Avantageusement, l'invention permet de réaliser un test de Beth-Vincent et un test de Simonin sur un même dispositif sans appareillage spécifique et sans modification préalable de l'échantillon de sang.

Selon un dernier aspect, l'invention est également relative à un kit pour l'identification et la détermination d'antigènes érythrocytaires de groupes sanguins et des anticorps plasmatiques correspondants.

Ce kit comprend au moins un dispositif tel que décrit précédemment, des réactifs anticorps permettant la détermination et l'identification des antigènes érythrocytaires des groupes sanguins, et des hématies tests permettant la détermination et l'identification des anticorps plasmatiques circulants.

## Revendications

1. Dispositif pour l'identification et la détermination de groupes sanguins à partir d'un échantillon de sang total, **caractérisé en ce qu'**il comprend un support solide (1) comportant au moins une zone réactive (4) sur laquelle le sang est déposé et sur laquelle le résultat est lu, ladite zone réactive étant constituée par au moins :
- une membrane poreuse (3) polymère en polyéthylène haute densité, présentant des pores de diamètre compris entre 1 et 20 µm, imprégnée par des réactifs anticorps monoclonaux ou des réactifs antiglobulines permettant l'immobilisation des anticorps plasmatiques, et
- une membrane absorbante (2), destinée à récupérer l'excès de sang de l'échantillon analysé.

2. Dispositif selon la revendication 1, **caractérisé en ce qu'**au moins une zone réactive (4) comprend également un filtre 5.

3. Dispositif selon la revendication 2, **caractérisé en ce que** le filtre (5) est constitué de fibres de verre de diamètre compris entre 1 et 7µm.

4. Dispositif selon la revendication 2 ou 3, **caractérisé en ce que** le filtre (5) est imprégné par des réactifs capables d'agglutiner les érythrocytes.

5. Dispositif selon l'une des précédentes revendications, **caractérisé en ce qu'**il comprend un réservoir 6 contenant une solution de lavage et/ou de révélation.

6. Utilisation du dispositif selon l'une des revendications 1 à 5, pour identifier et déterminer des groupes sanguins ABO, Rhésus et/ou RAI à partir d'un échantillon de sang total.

7. Procédé de phénotypage de groupes sanguins érythrocytaires, **caractérisé en ce qu'**il comprend les étapes suivantes :
- déposer un échantillon de sang total sur une zone réactive (4) d'un dispositif selon l'une des revendications 1 à 5,
- laisser réagir au moins 30 secondes, et
- appliquer une solution de lavage afin d'éluer les éléments non liés à la membrane poreuse (3).

8. Procédé de phénotypage de groupes ABO, Rhésus et/ou RAI, **caractérisé en ce qu'**il comprend les étapes suivantes :
- déposer un échantillon de sang total sur une zone réactive (4) d'un dispositif selon l'une des revendications 1 à 5, dont la membrane poreuse (3) a été imprégnée par des réactifs anticorps anti-antigènes spécifiques des érythrocytes,
- laisser réagir au moins 30 secondes, et
- appliquer une solution de lavage afin d'éluer les éléments non liés à la membrane poreuse (3).

9. Procédé de phénotypage de groupes ABO, Rhésus et/ou RAI, **caractérisé en ce qu'**il comprend les étapes suivantes :
- déposer un échantillon de sang total sur une zone réactive (4) d'un dispositif selon la revendication 4, dont la membrane poreuse (3) a été imprégnée par des réactifs antiglobulines permettant l'immobilisation des anticorps plasmatiques circulants,
- déposer des hématies-tests d'antigénicités connus sur ladite zone réactive (4),
- laisser agir au moins 30 secondes, et
- appliquer une solution de lavage afin d'éluer les éléments non liés à la membrane poreuse (3).

10. Kit pour la détermination et/ou la détection des groupes sanguins ABO, Rhésus et/ou RAI à partir d'un échantillon de sang total, **caractérisé en ce qu'**il comprend au moins :
- un dispositif selon l'une des revendications 1 à 5,
- des réactifs anticorps permettant la détermination et l'identification des antigènes érythrocytaires des groupes sanguins, et
- des hématies tests permettant la détermination et l'identification des anticorps plasmatiques circulants.

## Patentansprüche

1. Vorrichtung für die Identifikation und Bestimmung von Blutgruppen ausgehend von einer Gesamtblutprobe, **dadurch gekennzeichnet, dass** sie einen festen Träger (1) umfasst, der mindestens eine reaktive Zone (4) aufweist, auf der das Blut aufgetragen wird und auf der das Ergebnis abgelesen wird, wobei die reaktive Zone mindestens aus Folgendem besteht:
- eine poröse Polymermembran (3) aus Polyethylen hoher Dichte, die Poren mit einem Durchmesser zwischen 1 und 20 µm aufweist, imprägniert mit Reagenzien aus monoklonalen Antikörpern oder aus Antiglobulinen, welche die Immobilisierung der plasmatischen Antikörper ermöglichen, und
- eine Absorptionsmembran (2), die zum Auffangen des Überschusses an Blut der analysierten Probe bestimmt ist.

2. Vorrichtung nach Anspruch 1, **dadurch gekennzeichnet, dass** mindestens eine reaktive Zone (4) auch ein Filter (5) umfasst.

3. Vorrichtung nach Anspruch 2, **dadurch gekennzeichnet, dass** das Filter (5) aus Glasfasern mit einem Durchmesser zwischen 1 und 7 µm besteht.

4. Vorrichtung nach Anspruch 2 oder 3, **dadurch gekennzeichnet, dass** das Filter (5) mit Reagenzien imprägniert ist, welche zum Agglutinieren der Erythrozyten fähig sind.

5. Vorrichtung nach einem der vorhergehenden Ansprüche, **dadurch gekennzeichnet, dass** sie einen Behälter (6) umfasst, der eine Waschlösung und/oder eine Entwicklerlösung enthält.

6. Verwendung der Vorrichtung nach einem der Ansprüche 1 bis 5 zum Identifizieren und Bestimmen der AB0-, Rhesus- und/oder RAI-Blutgruppen ausgehend von einer Gesamtblutprobe.

7. Verfahren zur Phänotypisierung von Erythrozyten-Blutgruppen, **dadurch gekennzeichnet, dass** es die folgenden Schritte umfasst:
- Auftragen einer Gesamtblutprobe auf eine reaktive Zone (4) einer Vorrichtung gemäß einem der Ansprüche 1 bis 5,
- Reagierenlassen für mindestens 30 Sekunden, und
- Anwenden einer Waschlösung, um die nicht an die poröse Membran (3) gebundenen Elemente zu eluieren.

8. Verfahren zur Phänotypisierung von AB0-, Rhesus- und/oder RAI-Blutgruppen, **dadurch gekennzeichnet, dass** es die folgenden Schritte umfasst:
- Auftragen einer Gesamtblutprobe auf eine reaktive Zone (4) einer Vorrichtung gemäß einem der Ansprüche 1 bis 5, deren poröse Membran (3) mit Anti-Antigen-Antikörper-Reagenzien imprägniert wurde, welche für Erythrozyten spezifisch sind,
- Reagierenlassen für mindestens 30 Sekunden, und
- Anwenden einer Waschlösung, um die nicht an die poröse Membran (3) gebundenen Elemente zu eluieren.

9. Verfahren zur Phänotypisierung von AB0-, Rhesus- und/oder RAI-Blutgruppen, **dadurch gekennzeichnet, dass** es die folgenden Schritte umfasst:
- Auftragen einer Gesamtblutprobe auf eine reaktive Zone (4) einer Vorrichtung gemäß Anspruch 4, deren poröse Membran (3) mit Antiglobulin-Reagenzien imprägniert wurde, welche die Immobilisierung der zirkulierenden plasmatischen Antikörper erlauben,
- Auftragen von Test-Erythrozyten mit bekannter Antigenizität auf die reaktive Zone (4),
- Reagierenlassen für mindestens 30 Sekunden, und
- Anwenden einer Waschlösung, um die nicht an die poröse Membran (3) gebundenen Elemente zu eluieren.

10. Kit für die Bestimmung und/oder Erkennung der AB0-, Rhesus- und/oder RAI-Blutgruppen ausgehend von einer Gesamtblutprobe, **dadurch gekennzeichnet, dass** es mindestens umfasst:
- eine Vorrichtung gemäß einem der Ansprüche 1 bis 5,
- Antikörper-Reagenzien, welche die Bestimmung und Identifikation von Erythrozyten-Antigenen der Blutgruppen ermöglichen, und
- Test-Erythrozyten, welche die Bestimmung und Identifikation der zirkulierenden plasmatischen Antikörper ermöglichen.

## Claims

1. Device for identifying and determining blood groups using a whole blood sample, **characterised in that** it comprises a solid supporting member (1) comprising at least one reactive zone (4) whereon the blood is deposited and whereon the result is read, said reactive zone consisting of at least:
- one high-density polyethylene polymer porous membrane (3), having pores between 1 and 20 µm in diameter, impregnated with monoclonal antibody reagents or antiglobulin reagents suitable for immobilising plasma antibodies, and
- an absorbent membrane (2), intended to retrieve the excess blood from the sample analysed.

2. Device according to claim 1, **characterised in that** at least one reactive zone (4) also comprises a filter (5).

3. Device according to claim 2, **characterised in that** the filter (5) consists of glass fibres between 1 and 7 µm in diameter.

4. Device according to claim 2 or 3, **characterised in that** the filter (5) is impregnated with reagents capable of agglutinating erythrocytes.

5. Device according to any of the above claims, **characterised in that** it comprises a container (6) containing a washing and/or detection solution.

6. Use of the device according to any of claims 1 to 5, for identifying and determining ABO, Rhesus and/or IAT blood groups using a whole blood sample.

7. Method for phenotyping erythrocyte blood groups, **characterised in that** it comprises the following steps:
- depositing a whole blood sample onto a reactive zone (4) of a device according to any of claims 1 to 5,
- leaving to react for at least 30 seconds, and
- applying a washing solution so as to elute the elements not bound to the porous membrane (3).

8. Method for phenotyping ABO, Rhesus and/or IAT groups, **characterised in that** it comprises the following steps:
- depositing a whole blood sample onto a reactive zone (4) of a device according to any of claims 1 to 5, wherein the porous membrane (3) has been impregnated with erythrocyte-specific anti-antigen antibody reagents,
- leaving to react for at least 30 seconds, and
- applying a washing solution so as to elute the elements not bound to the porous membrane (3).

9. Method for phenotyping ABO, Rhesus and/or IAT groups, **characterised in that** it comprises the following steps:
- depositing a whole blood sample onto a reactive zone (4) of a device according to claim 4, wherein the porous membrane (3) has been impregnated with antiglobulin reagents suitable for immobilising circulating plasma antibodies,
- depositing test blood cells of known antigenicity onto said reactive zone (4),
- leaving to stand for at least 30 seconds, and
- applying a washing solution so as to elute the elements not bound to the porous membrane (3).

10. Kit for determining and/or detecting ABO, Rhesus and/or IAT blood groups using a whole blood sample, **characterised in that** it comprises at least:
- one device according to any of claims 1 to 5,
- antibody reagents suitable for determining and identifying erythrocyte antigens of blood groups, and
- test blood cells suitable for determining and identifying circulating plasma antibodies.
